(19) Europäisches Patentamt
European Patent Office
Office européen des brevets

(11) EP 3 383 503 B1

(12) EUROPEAN PATENT SPECIFICATION

(45) Date of publication and mention of the grant of the patent:
**27.07.2022 Bulletin 2022/30**

(21) Application number: **15821225.8**

(22) Date of filing: **17.12.2015**

(51) International Patent Classification (IPC):
***A61Q 11/02** (2006.01)* ***A61K 8/22** (2006.01)*
***A61K 8/38** (2006.01)* ***A61K 8/46** (2006.01)*
***A61K 8/49** (2006.01)*

(52) Cooperative Patent Classification (CPC):
**A61K 8/22; A61K 8/38; A61K 8/466; A61K 8/494; A61K 8/498; A61Q 11/02;** A61K 2800/88

(86) International application number:
**PCT/US2015/066282**

(87) International publication number:
**WO 2017/105455 (22.06.2017 Gazette 2017/25)**

# (54) HYDROGEN PEROXIDE BOOSTER SYSTEM FOR ENHANCED TEETH WHITENING

WASSERSTOFFPEROXIDVERSTÄRKERSYSTEM ZUR VERBESSERTEN ZAHNBLEICHUNG

SYSTÈME D'ACCÉLÉRATEUR POUR LE PEROXYDE D'HYDROGÈNE ASSURANT UN BLANCHIMENT DES DENTS AMÉLIORÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**10.10.2018 Bulletin 2018/41**

(73) Proprietor: **Colgate-Palmolive Company**
**New York, NY 10022 (US)**

(72) Inventors:
- **DOGO-ISONAGIE, Cajetan**
  **Mount Laurel, NJ 08054 (US)**
- **XU, Guofeng**
  **Plainsboro**
  **New Jersey 08536 (US)**

(74) Representative: **Wibbelmann, Jobst**
**Wuesthoff & Wuesthoff**
**Patentanwälte PartG mbB**
**Schweigerstrasse 2**
**81541 München (DE)**

(56) References cited:
**US-A1- 2005 281 773** **US-A1- 2006 171 906**
**US-A1- 2009 311 198** **US-A1- 2015 265 511**

- **Guan Wang: "Synthesis of Bleach Activators with Varying Cationic Groups", , 1 January 2012 (2012-01-01), XP055266673, Retrieved from the Internet: URL:http://repository.lib.ncsu.edu/ir/bitstream/1840.16/7695/1/etd.pdf [retrieved on 2016-04-19]**
- **DATABASE GNPD [Online] MINTEL; 1 November 2007 (2007-11-01), "Advanced Whitening Toothpaste", XP002756852, Database accession no. 820591**
- **DATABASE GNPD [Online] MINTEL; 1 October 2003 (2003-10-01), "Whitening Toothpaste", XP002756853, Database accession no. 10151698**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 383 503 B1

## Description

## BACKGROUND

Disclosed herein are novel compositions, such as tooth whitening compositions, that include a bleach activator for enhanced whitening.

In a mammal, a tooth is comprised of an inner dentin layer and an outer hard enamel layer that is the protective layer of the tooth. The enamel layer of a tooth is naturally an opaque white or slightly off-white color. It is the enamel layer that can become stained or discolored, although the dentin layer can also be stained which has been shown to happen naturally over time. The enamel layer of a tooth is composed of hydroxy apatite mineral crystals that create a somewhat porous surface. These hydroxyapatite crystals form microscopic hexagonal rods or prisms that make up the enamel surface. As a result, the surface of the enamel layer presents microscopic spaces or pores between the prisms. It is believed that this porous nature of the enamel layer is what allows staining agents and discoloring substances to permeate the enamel and discolor the tooth. These remaining substances can occupy the microscopic spaces and eventually alter the color of the tooth.

Many substances that a person confronts or comes in contact with on a daily basis can stain or reduce the whiteness of one's teeth. In particular, the foods, tobacco products, and fluids that one consumes tend to stain one's teeth. These products or substances tend to accumulate on the enamel layer of the tooth and form a pellicle film over the teeth.

These staining and discoloring substances can then permeate the enamel layer. This problem occurs gradually over many years, but imparts a noticeable discoloration of the enamel of one's teeth. So long as the discolored teeth are still healthy and do not pose any health risk or problem, a product or substance that would whiten the discolored teeth would be advantageous. It is also essential that a tooth whitening product that is to be used at home or in private by the consumer be safe and easy to use and be stable and retain its whitening efficacy during its storage on retail store shelves as well as over the period of use by the consumer.

Products and substances that are presently available to whiten teeth, known as tooth whitening compositions, may include a variety of different ingredients formulated into a liquid, paste or gel carrier. The primary active ingredient in such products and substances that provides whitening action, known as the whitening agent, is a peroxide which whitens teeth by oxidizing the organic matrix of teeth.

There is a need in the art to enhance whitening action over that of conventional tooth whiteners. Accordingly, there is a need in the art for improved tooth whitening compositions that can provide superior whitening benefit compared with current commercial products and for tooth whitening systems for storing and providing such improved tooth whitening compositions.

## BRIEF SUMMARY

The present invention concerns a tooth whitening composition comprising:

at least one whitening agent, wherein the at least one whitening agent is in the form of polyvinylpyrrolidone - hydrogen peroxide (PVP-$H_2O_2$) complex;
at least one bleach activator,
wherein the at least one bleach activator is selected from the group consisting of N- or O Acyl compounds, tetraacetylglycouril, N-acetyl N-[4-(triethylammoniomethyl) benzoyl] caprolactam chloride, sodium acetoxy-benzene sulfonate, sodium-benzoyloxy benzene sulfonate, sodium-lauroyloxy-benzene sulfonate, sodiumisononanoyloxy benzene sulfonate, tetra acetyl ethylenediamine and sodium nonanoyloxybenzenesulfonate;
at least one abrasive; and
at least one anticalculus agent.

Further embodiments of the present invention are set forth in the appended claims.

## DETAILED DESCRIPTION

As used throughout, ranges are used as shorthand for describing each and every value that is within the range. Any value within the range can be selected as the terminus of the range. In addition, all references cited herein are hereby incorporated by referenced in their entireties. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

In some embodiments, the tooth whitening compositions, first components, second components, bleach activator and whitening agent disclosed herein are viscous liquids, such as gels, thereby enabling the product to be applied to the tooth surface, such as with a soft applicator pen or brush, or a mouth-tray. In an embodiment, at least one of the whitening agent and the bleach activator may be combined with an orally acceptable vehicle, and then further combined with each other to form a tooth whitening composition which may include a dentifrice. Such dentifrices may include a dental tablet, toothpaste (dental cream), tooth powders, or gel, or any other form known to one of skill in the art. As used herein, an "orally acceptable vehicle" refers to a material or combination of materials that are safe for use in the compositions of the present disclosure, commensurate with a reasonable benefit/risk ratio, with which the whitening agent, and other desired active ingredients may be associated while retaining significant efficacy.

In some embodiments, the tooth whitening composition disclosed herein is substantially anhydrous, meaning that substantially no water is added. The tooth whitening composition of embodiments may comprise trace levels of water from ingredients or from product manufacture; however, such trace levels are insubstantial and do not interfere with the stability of the tooth whitening composition. In an embodiment, the vehicle is a low water content orally acceptable vehicle and may include any known ingredients or additives. For example, the vehicle may include liquid mixtures of glycerin, and sorbitol.

The tooth whitening composition may be formed by mixing a whitening agent and a bleach activator. The whitening agent may be a first component and the bleach activator may be a second component of a multi-component tooth whitening system, such as the multi-component tooth whitening system described herein. In the method, the whitening agent and the bleach activator may be mixed, for example, by combining the whitening agent and the bleach activator provided from their separate storage chambers, to form the tooth whitening composition. In an example, the whitening agent and the bleach activator are mixed in or on an applicator such as a mouth-tray.

As further detailed below, the whitening agent and the bleach activator may be stored separately until time of use, such as until mixing them together to form the tooth whitening composition. The separation of the two components, that is, the whitening agent and the bleach activator disposed in separate storage chambers, until time of use, such as until they are mixed to forth the tooth whitening composition, provides for increased stability of each of the whitening agent peroxide and bleach activator.

The tooth whitening compositions described herein may include a first component, such as at least one whitening agent, and a second component, such as at least one bleach activator. The bleach activator may comprise an activated ester. Each of the first and the second components may further comprise other additional ingredients that include those known to one of skill in the art, including one or more of the following components: fluoride ion sources, surfactants, flavoring agents, sweeteners, desensitizing agents, antimicrobial agents, anti-caries agents, anti-calculus agents, tartar control agents, anti-inflammatory agents, buffering agents, vitamins, pigments and coloring agents, preservatives, and enzymes, as will be discussed in greater detail below. The buffering agents may be added with the whitening agent and/or with the bleach activator in their respective separate storage chambers, or via an additional storage chamber, such that mixing the first component and the second component forms a tooth whitening composition that comprises an appropriate pH, such as an alkaline pH, for example, for maximum reaction between the whitening agent and the bleach activator. In an embodiment, at least one of a buffering agent may be added to the whitening agent and/or the bleach activator in a first one of a storage chamber and a second one of a storage chamber, respectively, and the at least one of a buffering agent included with whitening agent in the first storage chamber may be different than the at least one of a buffering agent added with the bleach activator in the second storage chamber. Any one or more of these additional ingredients may be included in the first component that includes the tooth whitening agent and/or in the second component that includes the bleach activator. The same or different one or more of these additional ingredients may be included in the first component than in the second component.

*Whitening Agent*

In various embodiments, the tooth whitening compositions disclosed herein comprise at least one whitening agent as a main active ingredient. As further discussed below, a "whitening agent" is a material which effects whitening of a tooth surface to which it is applied.

The at least one whitening agent included in the tooth whitening composition is in the form of polyvinylpyrrolidone-$H_2O_2$ complexes (hereinafter "PVP-$H_2O_2$") Polyvinylpyrrolidone is also known as poly-N-vinyl-poly-2-pyrrolidone and commonly abbreviated to "PVP". PVP generally refers to a polymer containing vinylpyrrolidone (also referred to as N-vinylpyrrolidone, N-vinyl-2-pyrrolidione and N-vinyl-2-pyrrolidinone) as a monomeric unit. The monomeric unit consists of a polar amide group, four non-polar methylene groups and a non-polar methane group.

Both linear and cross-linked complexes of PVP-$H_2O_2$ are known in the art, and PVP-$H_2O_2$ is considered to be stable in an anhydrous environment. Upon exposure to highly aqueous environments, such as in the oral cavity, the PVP-$H_2O_2$ dissociates into individual species (PVP polymer and $H_2O_2$). In one embodiment, the PVP-$H_2O_2$ complex is 80% by weight polyvinylpyrrolidone and 20% by weight $H_2O_2$.

In an embodiment, the tooth whitening composition of the embodiments includes 1% of the whitening agent, which is in the form of the PVP-$H_2O_2$ complex.

Additionally, at least one stabilizer may be present with a composition that includes the whitening agent. Acetanilide or a similar organic material can also be used with a pyrophosphate stabilizer such as sodium acid pyrophosphate, present in an amount ranging from about 0.1% to about 1.0%, such as about 0.5%.

In certain embodiments, the tooth whitening composition may further comprise at least one agent to enhance release of the peroxide in the oral cavity as a part of the peroxide component whitening agent. In an embodiment, the at least one agent to enhance release of the peroxide of in the oral cavity may be provided with the tooth whitening agent in a first container, with the bleach activator in the second container or in a third container, the contents of which are mixed with the whitening agent and/or bleach activator upon use of the tooth whitening composition. Polypore, which is an allyl methacrylate crosspolymer, available from Amcol health & Beauty Solutions, Inc., is an exemplary enhancing agent.

In various embodiments, the at least one whitening agent is present in the tooth whitening composition in an amount of up to about 35%, including from about 0.035% to 17.5%, such as an amount less than or equal to about 10% and greater than about 0%, including from about 0% to about 0.1%, from about 0.1% to about 10%, or from about 0.1% to about 6%, including an amount of about 1% by weight relative to the total weight of the tooth whitening composition. Therefore, the concentration of tooth whitening agent in a first component disposed in a first storage chamber may be preselected and/or a volume of the first component may be controlled upon discharging from the first storage chamber such that the above amounts of whitening agent are provided in a tooth whitening composition formed by combining the tooth whitening agent and the bleach activator.

### *Bleach Activator*

In an embodiment, the tooth whitening composition-which may be anhydrousmay be exposed to an aqueous environment such as during application of the tooth whitening composition in the mouth.

In an embodiment, the tooth whitening composition may thus be formed by combining a first component that may include a whitening agent and a second component that may include a bleach activator. In order to keep each of the whitening agent and bleach activator stable, or to keep them from reacting with one another, they may each be kept in separate storage containers until time of use at which point they are combined to form a tooth whitening composition. However, for maximum reaction between the whitening agent and the bleach activator to form the tooth whitening composition, the first component, the second component or both the first component and second component may individually include at least one buffering agent in addition to the whitening agent and the bleach activator, respectively. The whitening agent may also be provided as an aqueous composition. Accordingly, when the first component comprising the aqueous composition of the whitening agent (and possibly at least one of a first buffering agent) is combined with the second component comprising the bleach activator (and possibly at least one of a second buffering agent that may the same or different than the first buffering agent), the combination may be provided at an appropriate pH for maximizing the reaction product, such as the tooth whitening composition. Exposure to an additional aqueous environment, such as the oral cavity, may further provide for an appropriate pH for maximizing the reaction product after combining the two components and thereby increase whitening effect.

The bleach activator may react with the whitening agent at an appropriate pH, such as within the range of 3 pH to 12 pH. Exemplary pH values may include a pH of greater than about 5.5 pH and less than or equal to about 9 pH, for example in a range of about 5.5 pH to about 6 pH, including a pH of greater than about 6 pH and less than or equal to about 9 pH. In an embodiment, the bleach activator may react with the peroxide at a pH in the range of about 6 pH to about 8 pH, for example a pH of about 7.5 pH. While stored in, for example, a first storage chamber, the first component comprising the whitening agent may be kept at an acidic pH (i.e., less than about 7pH). Meanwhile the second composition comprising the bleach activator may be anhydrous and, therefore, may not be characterized based on a corresponding pH. However, the second component may also include a buffering agent that, upon mixing the first component and the second component to form a tooth whitening composition, provides for higher pH for the tooth whitening composition as compared to a pH of the first component stored in the first storage chamber.

The bleach activators include N- or O Acyl compounds, tetraacetylglycouril, N-acetyl N-[4-(triethylammoniomethyl) benzoyl] caprolactam chloride, sodium acetoxy-benzene sulfonate, sodium-benzoyloxy benzene sulfonate, sodium-lauroyloxy-benzene sulfonate, sodiumisononanoyloxy benzene sulfonate, acylated sugar derivatives, pentaglucose, tetra acetyl ethylenediamine and sodium nonanoyloxybenzenesulfonate (SNOBS or simply, NOBS). In an embodiment, the bleach activator may be FUTURECHEM® Nonanoic acid, sulfophenyl ester, sodium salt (available from FutureFuel Chemical Company of Batesville, AR).

In an example, the bleach activator comprises sodium nonanoyloxybenzenesulfonate and so the reaction with peroxide forms peroxynonanoic acid (also known as pernonanoic acid) (PNA) as represented by reaction Scheme 1:

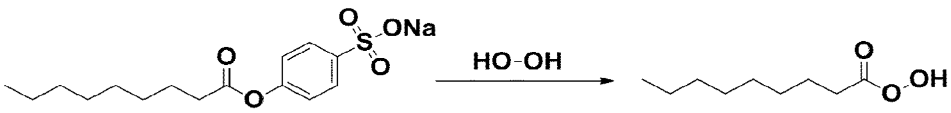


SNOBS Pernonanoic Acid Scheme 1

The bleach activator of the embodiments may be present in the tooth whitening composition in an amount ranging from about 0.1% to about 25% by weight, such as about 1.5% to about 5% by weight, for example about 2.5% by weight, relative to the total weight of the composition. Therefore, the concentration of bleach activator in a second component disposed in a second storage chamber may be preselected and/or a volume of the second component may be controlled upon discharging from the second storage chamber such that the above amounts of bleach activator are provided in a tooth whitening composition formed by combining the whitening agent and the bleach activator.

***Buffering Agent***

As discussed above, for maximum reaction between the whitening agent and the bleach activator to form the tooth whitening composition, the first component, the second component or both the first component and second component may individually include at least one buffering agent in addition to the whitening agent and the bleach activator. In an embodiment, the second component comprises the buffering agent. The buffering agent may be provided at whatever composition provides for a higher pH for the composition. The buffering agent may be the same or different than an anti- calculus agent, such as the materials described below for the anti-calculus agent of the embodiments. For example, the buffering agent may be one or more selected from TSPP, TKPP, STPP, sodium phosphate dibasic and sodium hydroxide.

***Additional Ingredients***

As previously described, many other ingredients may further be included in the whitening compositions of the present invention, and include surfactants, thickening agents, flavoring agents, sweetening agents, desensitizing agents, anti-microbial agents, anti-caries agents, anti-calculus agents, anti-inflammatory agents, vitamins, pigments and coloring agents, enzymes, preservatives, abrasive agents, and tartar control agents, for example. Any one or more of such additional ingredients may be included in the first component that includes the tooth whitening agent and/or in the second component that includes the bleach activator. The one or more additional ingredients that may be included in the first component may be the same or different ones of the one or more additional ingredients in the second component.

Any orally acceptable surfactant, most of which are anionic, nonionic or amphoteric, can be used. Suitable anionic surfactants include without limitation water-soluble salts of $C_{8-20}$ alkyl sulfates, sulfonated monoglycerides of $C_{8-20}$ fatty acids, sarcosinates, taurates and the like. Illustrative examples of these and other classes include sodium lauryl sulfate, sodium cocoyl monoglyceride sulfonate, sodium lauryl sarcosinate, sodium lauryl isoethionate, sodium laureth carboxylate and sodium dodecyl benzenesulfonate. Suitable nonionic surfactants include without limitation poloxamers, polyoxyethylene sorbitan esters, fatty alcohol ethoxylates, alkylphenol ethoxylates, tertiary amine oxides, tertiary phosphine oxides, dialkyl sulfoxides and the like. Suitable amphoteric surfactants include without limitation derivatives of $C_{8-20}$ aliphatic secondary and tertiary amines having an anionic group such as carboxylate, sulfate, sulfonate, phosphate or phosphonate. A suitable example is cocoamidopropyl betaine.

In some embodiments, one or more surfactants may be present in a total amount of from about 1.8% to about 2% w/w. In some embodiments, one or more surfactants may be present in a total amount of from about 1.9% to about 2% w/w. In some embodiments, one or more surfactants may be present in a total amount of about 2% w/w.

In some embodiments, the composition optionally comprises a thickening agent. Any orally acceptable thickening agent can be used, including without limitation carbomers, also known as carboxyvinyl polymers, carrageenans, also known as Irish moss and more particularly -carrageenan (iota-carrageenan), high molecular weight polyethylene glycols (such as CARBOWAX, available from The Dow Chemical Company), cellulosic polymers such as hydroxyethylcellulose, carboxymethylcellulose (CMC) and salts thereof, *e.g.,* CMC sodium, natural gums such as karaya, xanthan, gum arabic and tragacanth, colloidal magnesium aluminum silicate, and colloidal and/or fumed silica and mixtures of the same. In some embodiments, the one or more optional thickening agents are present in a total amount of about 0.1 % to about 90% w/w. In some embodiments, the one or more optional thickening agents are present in a total amount of about 1% to about 50% w/w. In some embodiments, the one or more optional thickening agents are present in a total amount of about 5% to about 35% w/w.

In certain embodiments disclosed herein, the tooth whitening composition may further comprise at least one flavoring agent. As an example, at least one flavoring agent may be included in the first component that includes the

tooth whitening agent and/or in the second component that includes the bleach activator. The same or different ones of an at least one flavoring agent may be included in the first component than in the second component.

The at least one flavoring agent, may, for example, be selected from essential oils, as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Of these, the most commonly employed are the oils of peppermint, spearmint and wintergreen. The flavoring agent may be incorporated in the tooth whitening compositions disclosed herein at a concentration ranging from 0.01% to about 2% by weight, such as about 0.1% to about 0.6% by weight.

In embodiments where the tooth whitening composition is sweetened, at least one sweetening agent may be used as an alternative or as a complement to the at least one flavoring agent. Suitable sweetening agents may be water-soluble and include, for example, sodium saccharin, sodium cyclamate, xylitol, perillartien, D-tryptophan, aspartame, dihydrochalcones and the like. The at least one sweetening agent may be present in the tooth whitening composition in an amount ranging from about 0.01% to about 1% by weight, such as about 0.3%.

Exemplary antimicrobial agents may include those typically used in oral care compositions, such as Thymol, benzyl alcohol, Triclosan, chlorhexidine, stannous salts including copper-, zinc-and stannous salts such as zinc oxide, zinc lactate, zinc citrate, zinc sulfate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2'methylenebis-(4-chloro-6-bromophenol).

The tooth whitening composition of the invention comprises a dental abrasive or combination of dental abrasive agents. As used herein, the term "abrasive" or "abrasive agent" also includes materials commonly referred to as "polishing agents." Any orally acceptable abrasive can be used, but typically, type, fineness (particle size) and amount of abrasive should be selected so that tooth enamel is not excessively abraded in normal use of the composition. Suitable abrasives include without limitation silica (in the form of silica gel, hydrated silica or precipitated silica), alumina, insoluble phosphates, calcium carbonate, resinous abrasives such as urea-formaldehyde condensation products and the like.

Among insoluble phosphates useful as abrasives are orthophosphates, polymetaphosphates and pyrophosphates. Illustrative examples are dicalcium orthophosphate dihydrate, calcium pyrophosphate, n-calcium pyrophosphate, tricalcium phosphate, calcium polymetaphosphate and insoluble sodium polymetaphosphate.

Average particle size of an abrasive, if present, is generally about 0.1 to about 30 $\mu$m for example about 1 to about 20 $\mu$m or about 5 to about 15 $\mu$m. In some embodiments, one or more abrasives are present in an amount of about 0.1 % to about 40% w/w. In some embodiments, the abrasive is calcium pyrophosphate. In some embodiments, the calcium pyrophosphate is present in an amount from about 5% to about 50% w/w.

The stable whitening dentifrice composition further comprises an anticalculus agent. Suitable anticalculus agents include without limitation phosphates and polyphosphates (for example pyrophosphates), polyaminopropanesulfonic acid (AMPS), hexametaphosphate salts, zinc citrate trihydrate, polypeptides, polyolefin sulfonates, polyolefin phosphates, diphosphonates. In some embodiments, the anticalculus agent is present in an amount of about 0.1% to about 30% w/w. In some embodiments, the stable whitening dentifrice composition comprises a mixture of anticalculus agents. In some embodiments, tetrapotassium pyrophosphate (TKPP), tetrasodium pyrophosphate (TSPP) and sodium tripolyphosphate (STPP) are used as the anticalculus agents. In some embodiments, the anticalculus agent comprises about 1% to about 2% w/w TSPP, and about 0% to about 7% w/w STPP.

Another desirable component of the present compositions is a synthetic anionic polymeric polycarboxylate (SAPP), which acts as a stabilizer for the polyphosphate anti-tartar agent and may help to block access of painful or pain-causing materials, such as sugars, to the tooth nerves.

Exemplary anti-inflammatory agents may include those typically used in oral care compositions, such as ibuprofen, flurbiprofen, aspirin, and indomethacine. Exemplary anti-caries agents may include ingredients such as sodium-, calcium-, magnesium-and stannous fluoride, aminefluorides, disodium monofluorophosphate and sodium trimetaphosphate. Exemplary vitamins may include ingredients such as Vitamin C. Exemplary desensitizing agents may include ingredients such as potassium citrate, potassium chloride, potassium tartrate, potassium bicarbonate, potassium oxalate, potassium nitrate and strontium salts. Exemplary anti-calculus agents may include ingredients such as pyrophosphate salts including the mono, di, tri and tetra alkali metal and ammonium pyrophosphate and tripolyphosphate salts. Exemplary enzymes may include glucoamylase.

Some embodiments provide compositions wherein at least one of ingredients is a fluoride ion source selected from stannous fluoride, sodium fluoride, potassium fluoride, sodium monofluorophosphate, sodium fluorosilicate, ammonium fluorosilicate, amine fluoride, and ammonium fluoride.

Also disclosed herein are methods for whitening a surface of a tooth in an oral cavity of a human or other animal subject. One such method comprises (a) applying a tooth whitening composition as disclosed herein to the tooth surface to be whitened for a plurality of minutes per day; and (b) repeating step (a) for multiple days to thereby whiten the teeth. Also disclosed herein is a method for whitening a surface of a tooth comprising: applying, to the surface of the tooth, a

composition comprising at least one whitening agent and at least one bleach activator comprising an activated ester. Another such method comprises (a) combining a tooth whitening agent disposed in a first storage chamber with a bleach activator disposed in a second storage chamber to form a tooth whitening composition, (b) applying the tooth whitening composition as disclosed herein to the tooth surface to be whitened for a plurality of minutes per day; and (c) repeating steps (a)-(b) for multiple days to thereby whiten the teeth. Also disclosed herein is a method for whitening a surface of a tooth comprising: combining a tooth whitening agent disposed in a first storage chamber with a bleach activator disposed in a second storage chamber to form a tooth whitening composition; applying, to the surface of the tooth, the tooth whitening composition comprising at least one whitening agent and at least one bleach activator

Exemplary methods disclosed herein comprise contacting the tooth whitening composition with the surface of the tooth. The contacting may occur for a duration of time sufficient to satisfactorily affect whitening of the teeth. Thus, the contacting occurs for a sufficient period of time to at least partially whiten teeth. This can be a period of time ranging from about 1 minute to about 2 hours or longer. In certain embodiments, the contacting is for a period of time ranging from about 1 minute to about 5 minutes, from about 1 minute to about 45 minutes, from about 5 minutes to about 45 minutes, or from about 5 minutes to about 30 minutes.

In certain embodiments disclosed herein, a tooth whitening composition, which may be substantially non-aqueous or may be aqueous, may be effective over a longer period of time, since it is not significantly diluted or washed away in the oral cavity during the treatment time. The tooth whitening composition can be removed as and when required, at will, by an employment of standard oral hygiene procedures such as brushing or by rinsing with an alcoholic mouthwash. While in place, the composition may release agents contained therein at a slow, relatively constant rate and in concentration sufficient to effect stain removal from or whitening of the teeth.

Further disclosed herein are methods of making a tooth whitening composition. In an example, a tooth whitening composition of an embodiment may be prepared by adding and mixing the ingredients of the composition in a suitable vessel, such as a stainless steel tank provided with a mixer. In the preparation of the tooth whitening composition, the ingredients may be added to the mixer in the following order: hydrophobic polymer component, such as the silicone based pressure sensitive polymer; peroxide whitening agent; adhesion enhancing agents, including fumed silica and petrolatum; and any desired flavoring or sweetener. The ingredients may then be mixed to form a homogenous dispersion/solution. In another example, a tooth whitening composition of an embodiment may be prepared by adding and mixing the ingredients of each of a first component including a whitening agent and/or the ingredients of a second component including a bleaching agent separately in a suitable vessel, such as a stainless steel tank provided with a mixer. In the preparation of the first component including a whitening agent and/or the second component including a bleach activator, the ingredients of each of the first component and the second component may be separately added to a mixer: i) for the first component: hydrophobic polymer component, such as the silicone based pressure sensitive polymer; peroxide whitening agent; adhesion enhancing agents, including fumed silica and petrolatum; and any desired flavoring or sweetener; and ii) for the second component: hydrophobic polymer component, such as the silicone based pressure sensitive polymer, bleach activator, adhesion enhancing agents, including fumed silica and petrolatum; and any desired flavoring or sweetener. The ingredients of each of the first component and in the second component may then be separately mixed to form a homogenous dispersion/solution for the first component and/or the second component.

A tooth whitening composition of an embodiment comprising at least one whitening agent and at least one bleach activator, for example, a first component comprising a whitening agent, the second component comprising a bleach activator wherein tooth whitening composition formed by combining the first component and the second component, may be prepared in the form of a flowable viscous liquid dispersion, such as a gel. In an example, is the tooth whitening composition may be applied directly onto the user's teeth such as by painting the teeth with a soft applicator brush or applying by a mouth-tray, including placing the first component and the second component on the mouth tray and then inserting into the user's mouth. Application by the user leaves a coating of the composition on the teeth. Contact with an aqueous environment at the proper pH, such as water in an aqueous composition for the first component comprising the whitening agent, or such as saliva in the user's mouth, causes the at least one whitening agent, for example, $H_2O_2$, to dissociate and react with the at least one bleach activator, providing prolonged whitening treatment of the tooth sites.

## EXAMPLES

### Reference **Example 1** - **Conventional Tooth Whitening Composition (12% Hydrogen Peroxide, 0% Sodium nonanoyloxybenzenesulfonate)**

A conventional tooth-whitening composition having 12% hydrogen peroxide whitening agent was prepared without any bleach activator of the embodiments by mixing the components of Table 1, below. The resulting formulation, including a listing of ingredients listed by wt% is provided in Table 1.

TABLE 1

| INGREDIENT | Weight % |
|---|---|
| Thickener | 3 |
| Potassium Nitrate | 3 |
| Water and minors (color, fragrance, sweetener, flavor) | Q.S. |
| Humectant | 30 |
| 35% Hydrogen Peroxide | 34.29 |
| Sodium Hydroxide | 1.85 |

Reference **Example 2** - **Preparation of Compositions with 5% bleach activator**

To assess the ability of SNOBS to enhance the whitening efficacy of hydrogen peroxide, a first SNOBS-based composition comprising bleach activator sodium nonanoyloxybenzenesulfonate was prepared according to the composition of Table 2.

TABLE 2

| INGREDIENT | Weight % |
|---|---|
| Non-ionic surfactant | 45.5 |
| Humectant | 22. 5 |
| Thickening agents | 12 |
| Sodium tripolyphosphate, tetra sodium polyphosphate | 15 |
| SNOBS | 5 |

Reference **Example 3 -Forming a Mixed Gel Composition Comprising Whitening Agent and Bleach Activator (6% Whitening Agent; 2.5% Bleach Activator)**

A mixed gel composition was prepared by combining equal amounts of an aqueous 12% hydrogen peroxide gel with the 5% SNOBS gel to form a composition having a concentration after mixing of 6% hydrogen peroxide and 2.5% SNOBS. Example 4 - Measuring peracid concentration versus time.

Reference **Example 4** - **Peracid Generation**

To quantify the amount of pernonanoic acid (PNA) generated by mixing the peroxide gel and SNOBS gel, the Karst assay (Karst et al., Anal. Chem. 1997, 69, 3623-3627) was utilized. Briefly, equal parts of 12% hydrogen peroxide gel and 5% SNOBS gel were mixed. 20 $\mu$l of the mixture was added to a phosphoric acid solution at timed intervals to stop the generation of peracid, the results of which are summarized in Table 3. This solution was then allowed to react with an excess of methyl-p-tolylsulfide (MTS) to generate methyl-p-tolylsulfoxide (MTSO). MTSO is then quantified by HPLCand the concentration of PNA is back calculated. Table 4 shows the concentration of PNA generated.

TABLE 3

| Time / min | 0 | 2 | 5 | 15 | 30 |
|---|---|---|---|---|---|
| % PNA | 0 | 0.32 | 0.53 | 0.43 | 0.59 |

Results show that a significant amount( 0.6% at 30 minutes) of pernonanoic acid generated by mixing SNOBS gel with hydrogen peroxide gel. Peracid are better oxidants than hydrogen peroxide and thus results in imporoved whitening.

Reference **Example 5** --- **In vitro bovine enamel testing**

Bovine teeth (mounted on acrylic resin) were brushed with an abrasive toothpaste to achieve baseline L values between 60 and 65. Eight bovine teeth were separated into two groups and baseline L* a* b* values were recorded using a hand held spectrophotometer. Each tooth was placed in a 24 well plate and soaked in artificial saliva for 30 minutes. 100 $\mu$g of the mixed gel composition of Example 2 was spread around the surface of the bovine teeth and left for 30 minutes. The gel was then wiped from the bovine teeth surface.

L a b values for the bovine teeth were gathered. These values were used to calculate the change in whiteness for each bovine tooth after treatment as compared to baseline ($W^*_{baseline}$). The bovine teeth were then soaked in artificial saliva for 20 minutes. The process was repeated 7 times to mimic once-per-day use for one week. The calculated change in whiteness is reported here based on a W* value, which is a measure of the overall color change relative to pure white. It should be noted that the more negative value of $\Delta W^*$, the closer the tooth color is to white, where W* is determined as:

$$W^* = ((a^*-0)^2 + (b^*-0)^2 + (L^* - 100)^2)^{1/2}$$

and

$$\Delta W^* = W^*_{treated} - W^*_{baseline}.$$

Whitening efficacy was tested using the sample having 2.5 wt% sodium nonanoyloxybenzenesulfonate (SNOBS) as compared to a sample having 0 wt% SNOBS. After 7 treatments on bovine teeth, significant differences in whitening efficacy were obtained. The modified tooth-whitening composition containing 2.5 % SNOBS delivered superior whitening at faster rate compared to the composition containing hydrogen peroxide alone. As shown in Table 4, seven treatments with 6% HP has the equivalent result as approximately 3 treatments in the presence of the 2.5% SNOBS formulation.

Table 4 summarizes increased whitening ($\Delta W^*$) effect of bovine tooth samples treated with the 2.5 wt % of SNOBS-containing composition paste versus the control whitening paste with 6% hydrogen peroxide (0% SNOBS) over the course of 7 treatments.

TABLE 4

| Treatment | **1** | **2** | **3** | **4** | **5** | **6** | **7** |
|---|---|---|---|---|---|---|---|
| **6%HP** | -5.55 | -10.73 | -14.14 | -16.10 | -16.722 | -17.65 | -18.18 |
| **6% HP & 2.5% SNOBS** | -10.24 | -16.14 | -17.90 | -19.48 | -19.76 | -19.95 | -21.28 |

Reference **Example 6 - SNOBS Stability**

SNOBS was included an anhydrous formulation according to a composition based on the ingredients shown in Table 5.

TABLE 5

| **INGREDIENT** | % |
|---|---|
| Non-ionic Surfactant | 33 |
| Thickening agents | 40 |
| Humectant | 10 |
| Abrasives (fumed silica) | 5 |
| TSPP | 2 |
| SNOB S | 10 |

The composition of Table 5 was aged at 40°C and at room temperature for 8 weeks and 13 weeks respectively. The percentage of SNOBS remaining was quantified by HPLC. The results in Table 6 indicate that there is sufficient

SNOBS remaining in the composition after aging at both room temperature and at 40°C to be efficacious as a booster for the peroxide component in a tooth whitening composition.

TABLE 6

| Time / Aging Temp. | % SNOBS | % Loss of SNOBS |
|---|---|---|
| Initial | 9.6 | - |
| 4 weeks / 40 °C | 936 | 3.1 |
| 8 weeks / 40 °C | 8.99 | 6.4 |
| 1 3 weeks / Room Temperature (RT) | 9.21 | 4.06 |

**Example 7** - **Conventional Tooth Whitening Composition (0% Sodium nonanoyloxybenzenesulfonate)**

A conventional tooth-whitening toothpaste having 1% hydrogen peroxide whitening agent was prepared without any bleach activator by mixing the components of Table 1, below. The resulting formulation, including a listing of ingredients listed by wt% is provided in Table 7.

TABLE 7

| INGREDIENT | Weight % |
|---|---|
| Humectant | 44.16 |
| Abrasive System II | 18.38 |
| Thickening agents | 23.38 |
| Cross-linked PVP complexed with Hydrogen Peroxide | 5.50 |
| minors (color, fragrance, flavor) | Q.S. |
| Tetrasodium Pyrhophosphate - Fine FCC | 2.00 |
| Sodium Lauryl Sulfate Powder | 2.00 |
| Sodium Acid Pyrophosphate | 0.90 |
| Sodium Monofluorophosphate -USP | 0.76 |
| Butylated hydroxy toluene, FCC grade | 0.03 |
| Total | 100.00 |

**Example 8** - **Tooth Whitening Toothpaste with Bleach Activator (1% Whitening Agent; 2.5% Bleach Activator)**

Bleach activator sodium nonanoyloxybenzenesulfonate (SNOBS) was added to the commercial (1% hydrogen peroxide) tooth whitening formulation at 2.5% of Table 1 by decreasing the amount of propylene glycol based so as to arrive at the formulation listed in Table 8.

TABLE 8

| INGREDIENT | Weight % |
|---|---|
| Humectant | 41.66 |
| Abrasive System II | 18.38 |
| Thickening agents | 23.38 |
| Cross-linked PVP complexed with Hydrogen Peroxide | 5.500 |
| minors (color, fragrance, flavor) | Q.S. |
| Tetrasodium Pyrhophosphate - Fine FCC | 2.000 |
| Sodium Lauryl Sulfate Powder | 2.000 |

(continued)

| INGREDIENT | Weight % |
|---|---|
| Sodium Acid Pyrophosphate | 0.900 |
| Sodium Monofluorophosphate - USP | 0.760 |
| Butylated hydroxytoluene, FCC grade | 0.030 |
| Nonanoic acid, sulfophenyl ester, sodium salt (1:1) | 2.500 |
| Total | 100.000 |

**Example 9** - **In vitro bovine enamel testing**

Bovine teeth (mounted on acrylic resin) were brushed with an abrasive toothpaste to achieve baseline L values between 60 and 65. Bovine teeth were separated into 3 groups and baseline L* a* b* values were recorded using a hand held spectrophotometer. Each grouping of bovine teeth was placed in a designated glass beaker for subsequent treatment with toothpaste containing 0% (Conventional Tooth whitening formulation), 2.5% (Tooth whitening formulation of Table 8), and 5% SNOBS as shown in Table 9 (further addition of bleach activator to formulation of Table 8 while decreasing the amount of propylene glycol), respectively.

TABLE 9

| INGREDIENT | Weight % |
|---|---|
| Humectant | 39.16 |
| Abrasives | 18.38 |
| Thickening agents | 23.38 |
| Cross-linked PVP complexed with Hydrogen Peroxide | 5.50 |
| minors (color, fragrance, flavor) | Q.S. |
| Tetrasodium Pyrophosphate --- Fine FCC | 2.00 |
| Sodium Lauryl Sulfate Powder | 2.00 |
| Sodium Acid Pyrophosphate | 0.90 |
| Sodium Monofluorophosphate-USP | 0.76 |
| Butylated hydroxytoluene, FCC grade | 0.03 |
| Nonanoic acid, sulfophenyl ester, sodium salt (1:1) | 5.00 |
| Total | 100.00 |

Samples containing 7g of optic white toothpaste with 0 wt%, 2.5 wt% and 5 wt% of SNOBS bleach activator were separately prepared by mixing with 7ml of artificial saliva. The samples were quickly mixed and poured into the designated beakers containing the bovine teeth described above. The beakers were placed in a shaker and left to shake for 2 minutes. The bovine teeth from each beaker were then rinsed with water until the toothpaste was washed away. The shaking and rinsing steps were repeated 14 times to mimic one week of toothpaste usage (i.e., 14 treatments) for each of the beakers. L a b values for the bovine teeth were gathered after the $7^{th}$ and $14^{th}$ treatment ($W^*_{treatrnent}$). These values were used to calculate the change in whiteness for each bovine tooth after treatment as compared to baseline ($W^*_{bascline}$). The calculated change in whiteness is reported here based on a W* value, which is a measure of the overall color change relative to pure white. It should be noted that the more negative value of ΔW*, the closer the tooth color is to white, where W* is determined as:

$$W^* = ((a^*\text{-}0)^2 + (b^*\text{-}0)^2 + (L^* - 100)^2)^{1/2}$$

and

$$\Delta W^* = W^*_{treated} - W^*_{baseline}.$$

Whitening efficacy was tested using the sample having 2.5 wt% sodium nonanoyloxybenzenesulfonate (SNOBS) added to a conventional, commercial tooth-whitening toothpaste (1% hydrogen peroxide). After 14 two minute treatments on bovine teeth, significant differences in whitening efficacy were obtained. The modified tooth-whitening toothpaste containing 2.5 % SNOBS showed a 64% increase in whitening versus commercial tooth-whitening toothpaste that does not include any of the SNOBS as shown in Table 10. Overall, the data in Table 10 shows SNOBS is a promising candidate to boost whitening.

Table 10 summarizes increased whitening (ΔW*) effect of bovine tooth samples treated with 5% and 2.5 wt % of SNOBS-containing whitening paste versus a control whitening paste with (0% SNOBS).

TABLE 10

| | **7 Treatments** | **14 Treatments** |
|---|---|---|
| **0% SNOBS** | -1.14 | -2.38 |
| **2.5% SNOBS** | -2.66 | -3.91 |
| **5% SNOBS** | -2.31 | -4.37 |

**Example 10** - **Stability of Optic White (OW) with 2.5% SNOBS toothpaste in 60C Ageing**

Batches of a commercially available tooth-whitening system (Optic White 1% HP, available from Colgate-Palmolive) and a tooth-whitening system of the embodiments comprising 2.5 % sodium nonanoyloxybenzenesulfonate (SNOBS) bleach activator were placed in an oven at 60°C oven. Hydrogen peroxide content was measured initially, after 1 week, and after 2 weeks.

The amount of hydrogen peroxide remaining in each sample was determined by reacting hydrogen peroxide in the toothpaste with potassium iodide in an acid media to liberate iodine. The liberated iodine was then titrated with standard sodium thiosulfate via manual titration. The percent hydrogen peroxide was calculated with equation 1 as follows:

$$\% \text{ Peroxide} = [(A \times N \times C) / (D)] \times 100/ \text{ Sample Volume (mL)},$$

where A is the volume of sodium thiosulfate (in mL), N is Normality of Sodium Thiosulfate solution, C is meq wt of oxidizing agent (e.g.., milliequivalent weight of hydrogen peroxide in standard sodium thiosulfate titration - 0.01701), and D is the sample volume (in mL).

After 1 week, the comparative optic white (OW) formulation with no bleach activator showed a slight decrease in the amount of peroxide (from about 1.0% initially to about 0.91% at week 1), and almost no subsequent change at week 2 (from about 0.91% at week 1 to about 0.905% at week 2). Similarly, the exemplary OW formulation with 2.5% SNOBS showed an acceptable decrease in the amount of peroxide (from about 0.950% initially to about 0.800% at week 1), and an acceptable subsequent change at week 2 (from about 0.800% at week 1 to about 0.730% at week 2). This 60°C aging study shows that hydrogen peroxide can remain at acceptable levels, even in the presence of 2.5% SNOBS in the tooth whitening formulations of the embodiments.

## Claims

**1.** A tooth whitening composition, comprising:

at least one whitening agent, wherein the at least one whitening agent is in the form of polyvinylpyrrolidone - hydrogen peroxide (PVP-$H_2O_2$) complex;
at least one bleach activator,
wherein the at least one bleach activator is selected from the group consisting of N- or O Acyl compounds, tetraacetylglycouril, N-acetyl N-[4-(triethylammoniomethyl) benzoyl] caprolactam chloride, sodium acetoxy-benzene sulfonate, sodium-benzoyloxy benzene sulfonate, sodium-lauroyloxy-benzene sulfonate, sodiumisononanoyloxy benzene sulfonate, tetra acetyl ethylenediamine and sodium nonanoyloxybenzenesulfonate;
at least one abrasive; and

at least one anticalculus agent.

2. The composition of claim 1, wherein the at least one bleach activator is present in an amount greater than an amount of the at least one whitening agent.

3. The composition of any one of the preceding claims, wherein the at least one whitening agent is present in an amount of greater than about 0 wt% and less than about 10%.

4. The composition of any one of the preceding claims, wherein the at least one whitening agent is present in an amount of greater than about 2 wt% and less than about 6 wt %.

5. The composition of any one of the preceding claims, wherein the composition is substantially anhydrous, optionally wherein the composition is a gel.

6. The composition of any one of the preceding claims, wherein the abrasive agent is selected from the group consisting of silica, alumina, insoluble phosphate, calcium carbonate, resinous abrasive, and mixtures thereof.

7. The composition of any one of the preceding claims, wherein the anticalculus agent is selected from the group consisting of phosphate, polyphosphate, pyrophosphate, polyaminopropanesulfonic acid (AMPS), hexametaphosphate salt, zinc citrate trihydrate, polypeptide, polyolefin sulfonate, polyolefin phosphate, diphosphonate, and mixtures thereof.

8. The composition of any one of the preceding claims, wherein the at least one bleach activator is sodium nonanoyloxybenzenesulfonate.

9. A method for whitening a surface of a tooth comprising:
contacting the surface of the tooth with a tooth whitening composition according to any one of the preceding claims for a duration of time sufficient to effect whitening of the surface of the tooth.

10. The method of claim 9, wherein the duration of time ranges from about 1 minute to about 45 minutes.

11. The method of claim 9 or 10, wherein the at least one bleach activator is present in an amount greater than the amount of the at least one whitening agent.

12. The method of any one of the preceding claims 9 to 11, further comprising exposing the tooth whitening composition to an aqueous environment comprising a pH of about 6 to about 8 pH.

**Patentansprüche**

1. Zahnaufhellende Zusammensetzung, umfassend:

mindestens ein aufhellendes Mittel, wobei das mindestens eine aufhellende Mittel in der Form eines Polyvinylpyrrolidon-Wasserstoffperoxid (PVP-$H_2$-$O_2$) Komplexes vorliegt;
mindestens einen Bleichaktivator,
wobei der mindestens eine Bleichaktivator ausgewählt ist aus der Gruppe bestehend aus N- oder O-Acylverbindungen, Tetraacetylglycouril, N-Acetyl-N-[4-(triethylammoniomethyl)benzoyl]-caprolactamchlorid, Natrium-Acetoxy-Benzolsulfonat, Natrium-Benzoyloxy-Benzolsulfonat, Natrium-Lauroyloxy-Benzolsulfonat, Natrium-Isononanoyloxy-Benzolsulfonat, Tetraacetylethylendiamin und Natrium-Nonanoyloxy-Benzolsulfonat;
mindestens ein abrasives Mittel; und
mindestens ein Antizahnstein-Mittel.

2. Zusammensetzung nach Anspruch 1, wobei der mindestens eine Bleichaktivator in einer Menge vorhanden ist, die größer ist als die Menge des mindestens einen aufhellenden Mittels.

3. Zusammensetzung nach einem beliebigen der vorhergehenden Ansprüche, wobei das mindestens eine aufhellende Mittel in einer Menge von mehr als etwa 0 Gew.-% und weniger als etwa 10 % vorhanden ist.

4. Zusammensetzung nach einem beliebigen der vorhergehenden Ansprüche, wobei das mindestens eine aufhellende Mittel in einer Menge von mehr als etwa 2 Gew.-% und weniger als etwa 6 Gew.-% vorhanden ist.

5. Zusammensetzung nach einem beliebigen der vorhergehenden Ansprüche, wobei die Zusammensetzung im Wesentlichen wasserfrei ist, gegebenenfalls wobei die Zusammensetzung ein Gel ist.

6. Zusammensetzung nach einem beliebigen der vorhergehenden Ansprüche, wobei das abrasive Mittel ausgewählt ist aus der Gruppe bestehend aus Silica, Alumina, unlöslichem Phosphat, Calciumcarbonat, harzhaltigem abrasivem Mittel, und Mischungen davon.

7. Zusammensetzung nach einem beliebigen der vorhergehenden Ansprüche, wobei das Antizahnstein-Mittel ausgewählt ist aus der Gruppe bestehend aus Phosphat, Polyphosphat, Pyrophosphat, Polyaminopropansulfonsäure (AMPS), Hexametaphosphatsalz, Zinkcitrat-Trihydrat, Polypeptid, Polyolefinsulfonat, Polyolefinphosphat, Diphosphonat, und Mischungen davon.

8. Zusammensetzung nach einem beliebigen der vorhergehenden Ansprüche, wobei der mindestens eine Bleichaktivator Natrium-Nonanoyloxy-Benzolsulfonat ist.

9. Verfahren zum Aufhellen einer Zahnoberfläche umfassend:
Inkontaktbringen der Zahnoberfläche mit einer zahnaufhellenden Zusammensetzung gemäß einem beliebigen der vorhergehenden Ansprüche für eine Zeitdauer, ausreichend um eine Aufhellung der Zahnoberfläche zu bewirken.

10. Verfahren nach Anspruch 9, wobei die Zeitdauer im Bereich von etwa 1 Minute bis etwa 45 Minuten liegt.

11. Verfahren nach Anspruch 9 oder 10, wobei der mindestens eine Bleichaktivator in einer Menge vorhanden ist, die größer ist als die Menge des mindestens einen aufhellendes Mittels.

12. Verfahren nach einem beliebigen der vorhergehenden Ansprüche 9 bis 11, weiterhin umfassend Aussetzen der zahnaufhellenden Zusammensetzung einer wässrigen Umgebung umfassend einen pH-Wert von etwa 6 bis etwa 8.

**Revendications**

1. Composition de blanchiment des dents, comprenant :

   au moins un agent de blanchiment, dans lequel l'au moins un agent de blanchiment est sous la forme d'un complexe polyvinylpyrrolidone-peroxyde d'hydrogène (PVP-$H_2O_2$) ; au moins un activateur de blanchiment, dans laquelle l'au moins un activateur de blanchiment est choisi dans le groupe constitué par les composés N- ou O-acyle, le tétraacétylglycouril, le chlorure de N-acétyl N-[4-(triéthylammoniométhyl)benzoyl]caprolactame, l'acétoxy-benzène sulfonate de sodium, le benzoyloxy benzène sulfonate de sodium, le lauroyloxybenzène sulfonate de sodium, l'isononanoyloxy benzène sulfonate de sodium, la tétraacétyl éthylènediamine et le nonanoyloxybenzène sulfonate de sodium ;
   au moins un abrasif ; et
   au moins un agent antitartre.

2. Composition selon la revendication 1, dans laquelle l'au moins un activateur de blanchiment est présent en une quantité supérieure à une quantité de l'au moins un agent de blanchiment.

3. Composition selon l'une quelconque des revendications précédentes, dans laquelle l'au moins un agent de blanchiment est présent en une quantité supérieure à environ 0 % en poids et inférieure à environ 10 %.

4. Composition selon l'une quelconque des revendications précédentes, dans laquelle l'au moins un agent de blanchiment est présent en une quantité supérieure à environ 2 % en poids et inférieure à environ 6 % en poids.

5. Composition selon l'une quelconque des revendications précédentes, dans laquelle la composition est sensiblement anhydre, éventuellement dans laquelle la composition est un gel.

6. Composition selon l'une quelconque des revendications précédentes, dans laquelle l'agent abrasif est choisi dans

le groupe constitué par la silice, l'alumine, un phosphate insoluble, le carbonate de calcium, un abrasif résineux et leurs mélanges.

**7.** Composition selon l'une quelconque des revendications précédentes, dans laquelle l'agent antitartre est choisi dans le groupe constitué par un sel phosphate, polyphosphate, pyrophosphate, d'acide polyaminopropanesulfonique (AMPS), hexamétaphosphate, le citrate de zinc trihydraté, un polypeptide, un polyoléfine sulfonate, un polyoléfine phosphate, le diphosphonate et leurs mélanges.

**8.** Composition selon l'une quelconque des revendications précédentes, dans laquelle l'au moins un activateur de blanchiment est le nonanoyloxybenzènesulfonate de sodium.

**9.** Procédé pour blanchir une surface d'une dent comprenant :
la mise en contact de la surface de la dent avec une composition de blanchiment des dents selon l'une quelconque des revendications précédentes pendant une durée suffisante pour effectuer un blanchiment de la surface de la dent.

**10.** Procédé selon la revendication 9, dans lequel la durée varie d'environ 1 minute à environ 45 minutes.

**11.** Procédé selon la revendication 9 ou 10, dans lequel l'au moins un activateur de blanchiment est présent en une quantité supérieure à la quantité de l'au moins un agent de blanchiment.

**12.** Procédé selon l'une quelconque des revendications 9 à 11 précédentes, comprenant en outre l'exposition de la composition de blanchiment des dents à un environnement aqueux comprenant un pH d'environ 6 à un pH d'environ 8.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **KARST et al.** *Anal. Chem.,* 1997, vol. 69, 3623-3627